# EUROPEAN PATENT SPECIFICATION

(11) **EP 1 957 098 B1**
(45) Date of publication and mention of the grant of the patent: **02.09.2009**
(21) Application number: 07804504.4
(22) Date of filing: 03.09.2007
(51) Int. Cl.: A61K 38/48, A61K 31/79, A61K 33/18, A61P 17/02

(54) **MANAGEMENT OF NON SEPTIC PATIENTS SUFFERING FROM PARTIAL THICKNESS BURNS BY ADMINISTRATION OF ACTIVATED PROTEIN C**
BEHANDLUNG VON NICHT-SEPTISCHEN PATIENTEN MIT VERBRENNUNGEN ZWEITEN GRADES MITTELS VERABREICHUNG VON AKTIVIERTEM PROTEIN C
MODE DE TRAITEMENT DE PATIENTS NON SEPTIQUES SOUFFRANT DE BRÛLURES D'ÉPAISSEUR PARTIELLE PAR ADMINISTRATION CONTINUE DE PROTÉINE C

(30) Priority: 01.09.2006 GR 20060100494
(43) Date of publication of application: 20.08.2008
(73) Proprietor: Tsagarakis, Myron, 115 28 Illissia, Athens (GR); Tsoutsos, Dimonsthenes, 152 35 Vrillissia Attikis (GR); Papalois, Apostolos, 153 41 Paraskevi Attikis (GR)
(72) Inventor: Tsagarakis, Myron, 115 28 Illissia, Athens (GR); Tsoutsos, Dimonsthenes, 152 35 Vrillissia Attikis (GR); Papalois, Apostolos, 153 41 Paraskevi Attikis (GR)
(86) International application number: PCT/GR2007/000043
(87) International publication number: WO 2008/026014

(56) References cited:
- EP-A- 0 913 156
- WO-A-02/100445
- US-A- 4 301 145
- US-A- 4 401 651

## Description

### DETAILED DESCRIPTION OF THE INVENTION

The invention in particular relates to the management of burn injuries. It offers a new way of management of non septic patients suffering from partial thickness bums during the first 48 post burn hours with the continuous administration of activated protein C and the topical treatment with a bactericidal agent in order to reduce the total burn injury and thus speed up the rate of tissue regeneration during the first post burn week.

Burn injuries are among the worst traumas which can happen to man. The larger burn injury, the most severe the consequences and the higher the chance of an adverse outcome or even death. In the Netherlands each year 40,000 people visit a general practitioner for the treatment of a burn would and 1600 people require in hospital care primarily for burns.

Approximately 80 % of the burn accidents happen in or around the house, mainly in the kitchen. Scalds, usually due to hot water, are the most common cause of burns. Water at 60°C will create a deep dermal or full-thickness burn in three seconds, and at 70°C the same burn will occur in one second. The temperature of freshly brewed coffee from a percolator is generally about 80°C which is hot enough to cause full-thickness burn in less than one second.

Children are particularly at high risk to burns. Hot beverages, particularly coffee and tea, are the predominant cause of scald bums in children. One study showed that 81 % of the burn injuries in children' under the age of 5 were due to scalds. Cooking oil, when hot enough to use for cooking, may be in the range of 150 - 180°C and can consequently cause very severe burns.

Bums are estimated to affect >1.4 million people in the United States annually. Of this number, 54.000 patients require hospital admission, 16.000 of whom have injuries of such significance that care is best undertaken in burn center. House and structure fires are responsible for >70% of the yearly 5.400 burn-related deaths, of which three fourths result from smoke inhalation or asphyxiation and one fourth are due to burns.

However, these fires are responsible for only 4% of burn admissions. Injuries due to contact with flame or ignition of clothing are the most common cause of burn in adults, whereas scald bums are most common in children. The majority of patients sustain bums limited severity and extent (>80% of burns involve <20% of the body surface) that they can be treated on an outpatient basis.

There are 250 to 275 patients per million population per year who require hospital admission owing to the extent of their bums or to other complicating factors. Approximately one third of patients who required in - hospital care have major burn injury - as defined by the American Burn Association on the basis of burn size, causative agent, pre-existing disease, and associated injuries and should be treated in a tertiary care burn center.

Other causes of burns are fire, electricity, chemical substances and even sunshine. In the Netherlands, around 200 people die of burn incidents each year, mostly at the place of accident. The case fatality rate of scald injury is low, instead most deaths occur in residential fires, commonly caused by careless smoking, by arson or by defective or inappropriately used heating devices.

The skin consists of two morphologically different layers that are derived from two different germ layers. The more superficial layer, the epidermis, is as specialized epithelial tissue derived from surface ectoderm. The deeper and thicker layer, the dermis, is composed of vascular dense connective tissue derived from mesenchyme.

In recent years the concept of the epidermis has gradually been changing from that of an innocent bystander, that, in its strictest sense, protects the body from the loss of fluids and electrolytes and the penetration of harmful substances, into that of an active participant in several important processes.

The dermis is situated between the epidermis and the subcutaneous fat. It supports the epidermis structurally and nutritionally. Its thickness varies, being greatest in the palms and soles and the least in the eyelids. With aging the dermis becomes thinner and loses elasticity.

The dermis interdigitates with the epidermis, so that the upward projections of the dermis, the dermal papillae, interlock with downward ridges of the epidermis, the rete ridges. Like all connective tissue, the dermis has three components : cells, fibers and amorphous ground substance.

The bulk of the dermis consists of a network of fibers, principally collagen, but also reticulum and elastin, packed bundles. Those in the papillary dermis being finer than those in the deeper, reticular dermis. The amorphous ground substance of the dermis consists largely of two glycosaminoglycans : hyaluronic acid and dermatan sulfate, with smaller amounts of heparin and chondroitin sulfate.

The function of the ground substance is that it binds water, in order to allow nutrients, hormones and waste products to pass through the dermis. It is also a lubricant between the collagen and elastic fiber network during skin movement and it provides bulk, allowing the dermis to act as a shock absorber. The dermis also contains muscles, both smooth and striated, and vessels.

Blood vessels are not only necessary for feeding, but also for regulation of the body temperature. Besides that, blood vessels play a role in allowing transendothelial migration of immune cells, by expressing adhesion molecules that bind to receptor molecules on the immune cells. This transmigration process allows immune cells into the tissue to o their surveillance work.

Lymphatic vessels, beginning as blind-ended capillaries in the dermal papillae, pass to either the superficial lymphatic plexus in the papillary dermis, or to the deeper horizontal plexuses. The play a role in the water homeostasis of the dermal tissue and also in the recirculation of the immune cells.

Thermal energy is a manifestation of random molecular kinetic energy. This energy is easily transferred from high energy molecules to those with a lower energy status during contact, fro example in living tissues. Both the temperature and the time period for which this temperature is sustained determine the degree of damage to all cell. At temperatures between 40 and 44°C, various enzyme systems begin to malfunction, and early denaturation of protein occurs.

Cellular functions become impaired, one of which is the membrane Na+ pump. This results in a high intracellular Na+ concentration and concomitant swelling of the cell. As the temperature increases, damage accumulation outruns the cell's inherent repair mechanisms and leads to eventual necrosis. The production of oxygen free radicals is part of this damage process.

These highly reactive molecules are capable of promoting further cell membrane abnormalities, leading to cell death. If the heat source is suddenly with-drawn, damage accumulation will continue until the cooling process brings cells back down to a normal temperature range. Cooling determines the difference between cell survival and cell death.

As the temperature increases, protein coagulation takes place, which causes destruction of the protein architecture. New aberrant bonds are formed, creating macro-molecules not similar to the original structures. The cell necrosis is complete, usually beginning at the skin surface, where the heat energy was absorbed most directly, extending downward.

This zone is called the zone of coagulation. The zone of stasis lies deeper and peripheral to the zone of coagulation. In this zone the damage is less and most cells are initially viable. However, the blood flow becomes progressively impaired and finally stops. This development of ischemia results in necrosis of the already affected cells.

Peripheral to this zone lays the zone of hyperemia, which is characterized by minimal cellular injury and prominent vasodilatation with increased blood flow, due to vasoactive mediators that were produced as part of the inflammatory response. Complete cellular recovery usually happens from this zone up only when capillaries will grow back upward.

Bums can be divided into different categories, based on the depth level of the tissue damage. First degree burn injury involves damage only, to the epidermis and is rarely clinically significant other being painful. The involved area is initially erythematous due to vasodilatation. Eventually desquamation happens, but this is followed by complete scarless healing within 7 days.

Second degree bums are partial - thickness by definition and are further categorized into superficial and deep. In superficial injuries, the epidermis is destroyed as well as varying superficial portions of the dermis. These lesions are usually painful. Blistering is often present.

Healing generally occurs rapidly and completely through migration to the surface of epithelial stem cells which survive in deeper portions of the hair follicles as well as the sweat and sebaceous glands. Relatively little scarring occurs in a superficial injury, due to the limited inflammatory phase, which is cut short by wound closure (re-epithelialization) occurring within 2 weeks.

In deep partial - thickness wounds most of the dermis is destroyed and only in the deepest parts of the hair follicles, sweat and sebaceous glands few epithelial cells remain. As the epithelial cells have to emigrate from the depth, and due to the loss of stem cells, re-epithelializaion is greatly retarded in these wounds.

Heat kills the superficial nerve endings, so the wound is relatively insensitive. As the deeply situated pressure receptors may survive, pressure sensation can still be present. Blistering is usually absent due to the thicker adherent overlying eschar which prevents the lifting by the edema. Due to the long period of wound closure, the inflammatory phase is prolonged, which gives rise to extensive collagen deposition and consequently abundant scar formation.

In third degree or full-thickness bums necrosis of the entire thickness of the skin occurs. As these are no epithelial appendages left, healing can only occur by - repithelialization from the wound edges, or, in case of small wounds, by contraction of the wound edges. So third degree wounds are routinely treated with excision and skin grafting, serving as source of new stem cells.

As no nerve endings are left, this type of wound is insensitive. Infection, the risk of which is proportional to the extent of injury, continues to be the predominant determinant of outcome in thermally injured patients despite improvements in overall care in general and wound care in particular.

In particular, as a manifestation of the systemic immunosuppressive effects of burn injury, infection at other sites, predominantly in the lungs, remains the most typical cause of morbidity and death in these severely injured patients. Burn patients with or without inhalation injury commonly exhibit a clinical picture produced by systemic inflammation.

The phrase " systemic " inflammatory response syndrome ("SIRS") has been introduced to designate the signs and symptoms of patients suffering from such a condition. SIRS has a continuum of severity ranging from the presence of tachycardia, tachypnea, fever and leukocytosis, to refractory hypotension and, in its most severe form, shock and multiple organ system dysfunction.

In thermally injured patients, the most common cause of SIRS is the burn itself. Sepsis, SIRS with the presence of infection bacteremia, is also a common occurrence. Pathological alterations of metabolic, cardiovascular, gastrointestinal, and coagulation systems occur as a result of the hyperactive immune system.

Paradoxically, a state of immunosuppression often follows or co-exists with SIRS. The counter anti-inflammatory response syndrome (CARS) appears to be an adaptive mechanism designed to limit the injurious effects of systemic inflammation. However, this response may also render the host more susceptible to systemic infection due to impaired antimicrobial immunity.

Both cellular and humoral mechanisms are involved in these disease processes and have been extensively studied in various burn and sepsis models. The phrase systemic inflammatory response syndrome (SIRS) was recommended by the American College of Chest Physicians / Society for Critical Care Medicine (ACCP / SCCM) consensus conference in 1992 to describe a systemic inflammatory process, independent of its cause.

The proposal was based on clinical and experimental results indicating that a variety of conditions, both infectious and noninfectious (i.e. bums, ischemia-reperfusion injury, multiple trauma, pancreatitis), induce a similar host response. Two or more of the following conditions must be fulfilled for the diagnosis of SIRS to be made :
Body temperature > 38°C or <36°C
Heart rate > 90 beats / min
Respiratory rate >20/min or Paco2 <32 mmHg
Leukocyte count > 12.000 / 1 µ, < 4000 µL, or > 10% immature (band) forms.

All these path physiologic changes must occur as an acute alteration from baseline in the absence of other known causes for them such as chemotherapy - induced Neutrogena and leucopenia.

The control of invasive burn wound infection through the use of effective topical chemotherapy, prompt surgical excision, and timely closure of the burn wound has resulted in unsurpassed survival rates. Even so, infection remains the most common cause of death in these severely injured patients.

Changes in wound care over the past thirty years, including the use of effective topical antimicrobial chemotherapy and excision of the burned wound, have significantly reduced the occurrence of invasive burn wound infection and its related morbidity and mortality. Regular collection of cultures from patients permits early identification of the causative pathogens of those infections that do arise.

Moreover, infection control procedures, including strict enforcement of patient and staff hygiene and use of patient isolation methods, have been effective in controlling the spread of resistant organisms and eliminating them from the burn center. These advances and the improvements in general care of critically ill burn patients have resulted in markedly improved survival rates.

The invention provides a way for the management of burn injury in a subject believed to be in need thereof comprising providing the subject with activated protein C and a topical bactericidal agent as povidone iodine

In general, when treating bums patients, two, often conflicting needs of the patient need be met. For one the management of the affected, and locally seriously inflamed, skin deserves particular attention, on the other hand, the patient may also suffer from the consequences of a more systemic inflammatory response.

Thermal injury initiates a deleterious pathophysiologic response in every organ system, with the extent and duration of organ dysfunction proportionate to the size of the burn. Direct cellular damage is manifested by coagulation necrosis, with the depth of tissue destruction determined by the duration of contract and the temperature to which the tissue is exposed.

Following burn, the normal skin barrier to microbial penetration is lost, and the moist, protein-rich avascular eschar of the burn wound provides an excellent culture medium for microorganisms, that infect the burn injury.

Although the body logically responds to these infections by eliciting a (local) inflammation, the invention provides use of an activated prot-C solution and povidone iodine for the topical treatment of a burn wound in a subject, to actually counter the inflammation and prevent systemic responses and overly active scar tissue formation.

Wound management will vary according to the depth of the burn. The true depth of the burn will become more obvious with time and therefore the wound must be reassessed to ensure that wound management is appropriate. Systemic, and even topical, antibiotics are not to be used prophylactically, and are in general only appropriate when demonstrated infection is present.

The invention thus provides a method to treat a burn injury of a subject wherein the subject is provided with a topical agent directed against a bacterial infection such as bacteriostatic or bactericidal as povidone iodine.

While destruction of the mechanical barrier of the skin contributes to the increased susceptibility to infection, postburn alterations in immune function may also be of significant importance. Every component of the humoral and cellular limbs of the immune system appears to be affected after thermal injury, the magnitude and duration of dysfunction are proportional to the extent of injury.

Wound healing is the consequence of a continuous sequence of signals and responses in which epithelial, vascular, hemopoietic and connective tissue cells come together outside their usual domains, interact, repair the damage and having done so turn back to their normal functions.

The purpose of wound healing is to restore the functions of the skin, such as protection of the body against harmful environmental entities, prevention of entry of microorganisms and loss of plasma, the regulation of body temperature, the processing and interpretation of environmental information through the neurosensory system and a social - interactive function.

Vertical cutaneous injuries, such as surgical incisions which have a minimal loss of tissue, will essentially heal through the formation of a blood clot, rapid epithelialization, and fibroblast proliferation. Progressive collagenization and increased strength, which reach normal levels within weeks, will complete the healing process and leave discrete scarring, in most cases.

On the other hand, cutaneous wounds with a predominant horizontal loss of tissue, like burn injuries, exhibit a healing which proceeds through a series of complex, biological mechanisms according to the extent and level of the involved structures. In particular the destruction of the normal capillary system that is involved to the blood supply of the skin creates additional problems.

A burn wound that suffers from decreased blood supply becomes ischemic, hypoxic, and highly edematous. For various stages in the burn wound healing process use of a molecule according to the invention for the preparation of a pharmaceutical composition for modulation of vascularization or prevention of capillary network obstruction by coagulation in the Zone of Stasis (Zone II) and the Zone of Hyperaemia (Zone III) in wound repair, particularly in bums is herein provided

The wound healing response can be divided into three distinct, but overlapping phases : 1) hemostasis and inflammation, 2) dermal and epidermal proliferation, and 3) maturation and remodeling. The first response after disruption of tissue integrity, is to control the damage produced to the vascular system.

A hemorrhage means immediate danger to the body, which reacts with prompt vasoconstriction, platelet aggregation and activation of the coagulation system. The initial response to deep bums involves a transient 5- to 10- minute period of intense vasoconstriction that aids in hemostasis.

This is followed by active vasodilatation that usually becomes most pronounced approximately 20- minutes after the injury and is accompanied by an increased capillary permeability. Histamine is believed to be a key chemical mediator responsible for the vasodilatation and the danger in vascular permeability. Shortly after burning, platelet adhesion occurs at the site of the burn.

Platelets function to initiate the formation of a clot that helps to achieve hemostasis. The contact between the extracellular matrix and platelets, as well as the presence of thrombin and fibronectin, results in the release of growth factors and vasoactive substances such as platelet - derived growth factor (PDGF), transforming growth factor - β (TGF-β), fibroblast growth factor (FGF), epidermal growth factor (EGF), bradykinin, prostaglandins, prostacyclins, thromboxane, histamine and serotonin.

Platelet degranulation also initiates the complement cascade with the formation of C3a and C5a, which are potent anaphylatoxins promoting the release of histamine by basophiles and mast cells. When angiogenesis is promoted in a method as provided herein these series of events are accompanied by improved blood supply from regenerating tissue which ultimately leads to less complicated wound healing.

Also, granulocytes, in a rapid response to signaling by platelets and also through factors produced by the activation of the complement system, form the first line of defense against local bacterial contamination. In the absences of bacterial contamination, the granulocyte has been claimed to be non-essential to the wound healing process.

Usually within 24 - 72 hours, the granulocytes are gradually replaced by monocytes that acquire the characteristics of tissue macrophages and become central coordinators of the inflammatory and repair process. Macrophages not only help to clean the wounded area of undesirable debris and bacteria, but they also promote the built up of the new connective tissue.

Through growth factors and cytokines like TGF-β, PDGF and EGF, tumor necrosis factor - α (TNF-α), interleukin - 1 (IL-1) and interferon - gamma (IFN - gamma), through enzymes like collagenase and arginase, and through prostaglandins, they regulate the matrix synthesis by affecting either fibroblast chemotaxis or proliferation, or collagen synthesis.

Macrophages also play a role in mediating angiogenesis and in the recruitment and activation of other immune cells. Furthermore, it has been demonstrated that activated T lymphocytes, following the influx of granulocytes and macrophages, enter a wound area by day 4 or 5 and become important modulators of the healing process.

An intact T - cell immune system is essential, at least indirectly, for a normal healing outcome. Other cells, like mast cells, and their major protease, chymase, also play a role in the wound healing process by promoting capillary outgrowth and collagen formation. Again, these processes react well on treatment with activated Prot -C which prevents necrosis to spread to Zone II and Zone II of the burn injury.

It has also been suggested that dermal dendrite cells participate in wound repair by initiating the inflammatory response and by stimulating epithelial proliferation and restoration of epithelial architecture.

The crucial pathophysiologic event that precipitates systemic inflammation is tissue damage. This can occur both as a result of the direct injury to tissues from mechanical or thermal trauma as well as cellular injury induced by mediators of ischemia - reperfusion injury such as oxygen free radicals. Injury results in the acute release of proinflammatory cytokines.

If injury is severe, such as in extensive thermal injury, a profound release of cytokines occurs, resulting in the induction of a systemic inflammatory reaction, of which disseminated intravascular coagulation is often seen at on or more stages of the healing process.

The ability of the host to adapt to this systemic inflammatory response is dependent on the magnitude of the response, the duration of the response, and the adaptive capacity of the host. Factors that have been implicated in prolongation of SIRS include under resuscitation in the phase following thermal injury, persistent or intermittent infection, ongoing tissue necrosis, and translocation of endotoxin across the bowel.

In one embodiment, the invention is providing a method and means to treat the systemic reaction to bums injuries by providing a subject believed to be in need thereof with a pharmaceutical composition comprising down - regulating peptide or functional analogue thereof and an agent directed towards the protection of Zone II and Zone III from further cell damage and cellular death.

Such an agent may for example be a composition comprising heparin, however, in preferred embodiment the invention provides treatment with activated protein C, an agent used till now against disseminated intravascular coagulation, and different stages of sepsis.

Such an agent -not only to modulate disseminated intravascular coagulation (DIC)- but with a strong protective action on Zone II and Zone III of burn injuries comprises preferably (recombinant) human Activated Protein C.

### BURNS AND ACTIVATED PROTEIN C.

One of the major problems we face in the evaluation and management of burn victims is the gradual increase in tissue damage as far as depth and total body area of the affected skin is concerned. The direct skin damage due to the cause of burn (Zone I) often represents only 30% of the final damage to the tissues of the area.

This area is surrounded by a second zone were due to the release of toxic agents (hyper oxides, free radicals, lysosomes) ( Zawacki B. The local effects of burn injury p: 25-36. The art and science of burn care Boswick J. Jr. 1987 Aspen Publishers. Hinder F. and Traber D. Pathophysiology of the systemic inflammatory response syndrome. P: 207-217.

Total burn care Hemdon DN. 1996. W B Sauders) from the destroyed cells, venous stasis is observed which leads to the formation of thrombus in the microvessels and finally cellular death which result in an increase of tissue damage of more than 30% (Cook D. Pathophysiology of burns. P: 7-18. Bums trauma Bosworth C. 1997. Bailliere Tindall Bernard GR, Vincent JL, Lattere PF et al. N Engl J Med 2001;699-709)

A number of agents and substances have been used in an effort to preserve the blood flow in microcirculation mainly by preventing thrombosis. Those substances belong to anticoagulants and blood flow facilitators (such as heparine, urokinase, prostaglandin E1, magnesium sulphate, IIb/IIIa inhibitors of platelets) and others.

(Ichiro Hashimoto, MD., PhD., Hideki Nakanishi, M.D.Ph.D., Yoshitaka Shono, M.D.,Masahiro Yamano, M.D. and Maki Toda :THE FEATURES OF THROMBUS IN A MICROVESSEL INJURY MODEL AND THE ANTITHROMBOTIC EFFICACY OF HEPARIN,UROKINASE, AND PROSTAGLANDIN E1 2307-2314 Plast. And Reconstr. Surg. Vol 111 -7 June 2003)

Except for magnesium sulphate used in animal models for heart surgery in great vessels, the above stated substances when use in animal models of burn induced tissue damage, regardless of the cause of burn (electrical burn or thermal burn or radiation bum) had as an effect only the elongation of the period between the burn and the final tissue damage.

Tissue damage revealed to be the same as far as depth and extend was concerned. From these substances, heparin appears to be the most useful elongating the time from stasis to final thrombosis in both arterioles and venuoles.

(Ichiro Hashimoto,MD.,PhD.,Hideki Nakanishi,M.D.Ph.D.,Yoshitaka Shono, M.D.,Masahiro Yamano, M.D. and Maki Toda :THE FEATURES OF THROMBUS IN A MICROVESSEL INJURY MODEL AND THE ANTITHROMBOTIC EFFICACY OF HEPARIN,UROKINASE, AND PROSTAGLANDIN E1 2307-2314 Plast. And Reconstr. Surg. Vol 111 -7 June 2003)

Eventhouthg activated protein C is a very powerful anticoagulant, its use was never reported on early stages of burn injury (first 7 postburn days) neither in clinical nor experimental studies -up to now. As it is stated in EP 1 449 537 A2 (25.08.04) the latest patent related to uses of activated protein C "there is little data regarding whether protein C replacement therapy would be effective or regarding how to treat the patients with activated protein C.

This invention address exactly the above problem: A way to manage burn victims during the first 7 postburn days with the IV continuous infusion of activated protein C and a locally applied bactericidal agent (Povidone Iodine 10%) (Claim 1) And the use of activated protein C in the management of partial thickness burns as described in claim 1.

It has long been recognized that severely burned patients have complications associated with hypercoagulation (Curreri et al., Ann. Surg. 181:161-163 (1974). Burned patients have supranormal in vitro clothing activity and frequently develop DIC which is characterized by the sudden onset of diffuse haemorrhage, the consumption of fibrinogen, platelets, and Factor VII activity, intravascular haemolysis, secondary fibrinolysis and biopsy evidence of microthrombi (McManis, et al., J of Trauma 13:416-422,(1973).

Recently, it was reported that the levels of protein C were drastically reduced in severely burned patients and that this reduction of the natural anticoagulant may lead to an increase risk of bums (Ueyama, et al., Nippon Geka Gakkai Zasshi 92:907-12-1991). DIC is one of the common complications in patients suffering from severe burn injuries.

Based on the above facts a series of European and US patents, included the administration of activated protein C as a replacement therapy in the management of bums but clearly stating the lack of data to support the effectiveness and the way to administer such a treatment to burn victims.

Our invention based on sound experimental and clinical facts, establish the path physiological way through which activated protein C speeds up healing in partial thickness bums, and implements a protocol and way of administration of the above substance.

The way that activated protein C acts on early burn stages is not only by treating DIC which is mainly observed in sepsis and septic patients. Long before the patient becomes septic, during the first 48 post burn hours, when he is not yet colonized by harmful bacteria, the IV administration of activated protein C can exert most of its beneficial effect.

This is exerted directly in ZONE II (or Zone of Stasis) of the burn injury, by preventing coagulation, and decreasing cellular destruction by inflammation mechanisms in this area thus effectively stopping the progress of the burn injury, resulting in overall smaller volume of damaged tissue to be repaired.

The above mechanism of action is not described in any of the European and US patents which refer to activated protein C, (EP0913156 A1 19990506, US6156734A1 20001205, US6008199 A 19991228, EP1449537 A2 20040825, US2003224995 A1 20031204, US 2003/02111969 A1/ FISHER ET AL 13-11-2003, WO99/63070A1/ ELI LILLY & CO 9-12-1999) nor published in any state of the art medical journals, as there are no publication referring to the action of activated protein C in bums up to now.

The present invention discloses for the first time the use of activated protein C in a clinical trial in non-septic burned patients. In these patients the activated protein C treated group demonstrated statistical improvement in organ function, decreased mortality, and smaller hospitalization time as compared to the placebo group.

Thus the present invention establishes methods utilizing activated protein C in the treatment of partial thickness burns, thought the protective action of activated protein C in the survivability of the Zone of Stasis of bums if we administer the drug during the first 48 hours post burn.

The activated protein C can be formulated according to known method to prepare pharmaceutically useful compositions. The activated protein C will be administered parenterelly to ensure it's delivery into the blood stream in an effective form by injecting the appropriate dose as a continuous infusion for 48 hours. The amount of activated protein C administered will be about 24µg/kg/hr. (See tables with proposed doses)

### EXAMPLE 1

In our experimental study we will test the effectiveness of activated protein C, a new powerful antithrombotic agent, comparing her with the most useful -today- substance i.e. heparin, in the prevention of tissue damage to the zone of stasis (Zone II) in a hot iron induced thermal burn on an animal model. All experiments were carried out according to Act. 527/08/02/2006 of the Prefecture of East Attica in accordance to the 86/309/EU DIRECTIVE for animal protection.

### MATERIAL AND METHOD

Eighteen swines aged 3-4 months old and of an approximate weight of 10-12 kg were divided in 3 groups of 6 swines. We created 2 strips of partial thickness burns at the skin of the back of the pigs measuring approximately 3cm x 20 cm.

Then we create group A to which we administered activated protein C IV for 48 hours, group B which receives heparin IV and a third group which received only NaCL 0.9%. All the burned areas were treated topically with Povidone Iodine 10%.

### Stage I Creation of burn wound

For the creation of the burn wound we use an iron tip 5mmX 30mm preheated at 800°C. For the creation of the superficial partial thickness bums, the preheated tip was passed over the designated strips 5 times, while for the creation of the deep partial thickness burn the preheated tip was passed over the designated strips 10 times. All bums were created from the same doctor.

### Stage II Administration of drugs

Group a (Activated protein-C)
   Continuous IV administration for 48 hours at a dose of 24µg/Kg/hr+Povidone Iodine
Group B (Heparin)
   Bolus administration for 5min of 50 IU/Kgr + Povidone Iodine
Group C (Placebo)
   Administration of NaCl 0.9% 50ml/hr for 48 hours + Povidone Iodine

### EVALUATION OF RESULTS

For the evaluation of the results we shall used the classic way of histopathological evaluation of biopsies obtained at days 1, 3 and 7 i.e when burn wounds are generally in the first non septic phase.

The following features were studied: a) the zone of direct cellular necrosis resulting from direct thermal injury
b) The zone of tissue damage resulting from the thermal injury and from the toxins released from the dead cells
c) The overall size of Zone of cellular death +Zone of cellular injury.

After the creation of burn wound we obtain baseline readings from the affected and the non-affected areas of the skin. The following days (day 1, 3, 7) we obtain biopsies from the burned areas which were sent for histopathological examination. In this way we were able to effectively follow the dynamic changes, which took place in the three consecutive zones of burn.

Our results proved that in the first 7 post burn days the animal which received activated protein C healed 40% of their burn injury while the ones treated with heparin showed cellular damage 25% higher than the initial values as far as a partial thickness bums were concerned.

For deep partial thickness bums animals receiving activated protein C exhibited 21.6% healing while the ones treated with heparin showed cellular damage 34% higher than the initial values.

This clearly indicates that heparin, as reported in the literature(Ichiro Hashimoto,MD.,PhD.,Hideki Nakanishi,M.D.Ph.D., Yoshitaka Shono, M.D.,Masahiro Yamano, M.D. and Maki Toda :THE FEATURES OF THROMBUS IN A MICROVESSEL INJURY MODEL AND THE ANTITHROMBOTIC EFFICACY OF HEPARIN,UROKINASE, AND PROSTAGLANDIN E1 2307-2314 Plast. And Reconstr. Surg. Vol 111 -7 June 2003)
in the long run , does not protect ZONE II (Zone of stasis) from cellular damage, while activated Prot-C does protect this Zone from cellular damage ( Exact tables with measurements provided).

### STATISTICAL COMPARISON BETWEEN THE ACTION OF THE ADMINISTERED DRUGS IN THE DEPTH OF TOTAL BURN INJURY (ZONE I + ZONE II)

### PARTIAL THICKNESS BURN

Using the Kruskal-Wallis test we can examine the difference among the 3 therapies of percentage change from baseline to each time measurement of the depth of necrosis+Zone of cellular damage. Pairwise comparisons will be performed using the Mann-Whitney

| | Baseline-3^{rd} day | | | |
|---|---|---|---|---|
| | Median (%) | NaCl+Pov.Io | Heparine | Act-Prot. C |
| NaCl+Pov.Iod | 42.67 | --- | 0.026 | 0.002 |
| Heparine | -3.22 | --- | --- | 0.009 |
| Act.Prot C. | -25.64 | --- | --- | --- |
| Overall Sig. | 0.002 | | | |

There is statistically significant difference between the 3 therapies of percentage change from baseline to 3^{rd} day of the depth of necrosis+ Zone of cellular damage ( p=0,002 ) .Pair wise comparisons indicated statistically significant difference between all groups

| | Baseline-7^{th} day | | | |
|---|---|---|---|---|
| | Median (%) | NaCl+Pov.Io | Heparine | Act-Prot.C |
| NaCl+Pov.Io | -15.13 | --- | 0.026 | 0.026 |
| Heparine | 25.44 | --- | --- | 0.002 |
| Act.Prot.C | -40.56 | --- | --- | --- |
| Overall Sig. | 0.002 | | | |

There is statistically significant difference between the 3 therapies of percentage change from baseline to 7^{th} day of the depth of necrosis+ Zone of cellular damage ( p=0.002). Pair wise comparisons indicated statistically significant difference between all groups.

| | Baseline-13^{th} day | | | |
|---|---|---|---|---|
| | Median (%) | NaCl+Pov.Io | Heparine | Activ-Prot.C |
| NaCl+Pov.Io | -50.00 | --- | N.S | N.S |
| Heparine | 18.18 | --- | --- | 0.009 |
| Activ-Prot C | -53.03 | --- | --- | --- |
| Overall Sig. | 0.05 | | | |

There is statistically significant difference between the 3 therapies of percentage change from baseline to 13^{th} day of the depth of necrosis+ Zone of cellular damage ( p=0,05 ) . Pair wise comparisons indicated statistically significant difference between heparine and, activated Protein C

| | Baseline-16^{th} day | | | |
|---|---|---|---|---|
| | Median (%) | NaCl+Pov.Io | Heparine | Activ-Prot C |
| NaCl+Pov.Io | 24.62 | --- | N.S | 0.05 |
| Heparine | -6.06 | --- | --- | 0.06 |
| Activ-Prot C | -61.54 | --- | --- | --- |
| Overall Sig. | 0.003 | | | |

There is statistically significant difference between the 3 therapies of percentage change from baseline to 16^{th} day of the depth of necrosis+ Zone of cellular damage ( p=0,003 ) . Pair wise comparisons indicated statistically significant difference between activated Protein-C and heparin , Povidone Iodine respectively.

| | Baseline-20^{th} day | | | |
|---|---|---|---|---|
| | Median (%) | NaCl+Pov.Io | Heparine | Activ-Prot C |
| NaCl+Pov.Io | 53.13 | --- | 0.026 | 0.002 |
| Heparine | -17.80 | --- | --- | 0.015 |
| Activ-Prot C | -75.30 | --- | --- | --- |
| Overall Sig. | 0.002 | | | |

There is statistically significant difference between the 3 therapies of percentage change from baseline to 20^{th} day of the depth of necrosis+ Zone of cellular damage ( p=0,002 ) Pairwise comparisons indicated statistically significant difference between all groups

### STATISTICAL COMPARISON BETWEEN THE ACTION OF THE ADMINISTERED DRUGS IN THE DEPTH OF TOTAL BURN INJURY (ZONE I +ZONE II)

### DEEP PARTIAL THICKNESS BURN

Using the Kruskal-Wallis test we can examine the difference among the 3 therapies of percentage change from baseline to each time measurement of the width of necrosis+cellular injury. Pair wise comparisons will be performed using the Mann-Whitney

| | Baseline-3^{rd} day | | | |
|---|---|---|---|---|
| | Median (%) | NaCl+Pov.Io | Heparine | Activ-Prot C |
| NaCl+Pov.Io | 37.50 | --- | N.S | 0.002 |
| Heparine | 16.43 | --- | --- | 0.002 |
| Activ-Prot C | -26.14 | --- | --- | --- |
| Overall Sig. | 0.003 | | | |

There is statistically significant difference between the 3 therapies of percentage change from baseline to 3^{rd} day of the depth of necrosis+Zone of cellular damage ( p=0,003 ) . Pair wise comparisons indicated statistically significant difference between activated protein C and heparine, betadine respectively.

| | Baseline-7^{th} day | | | |
|---|---|---|---|---|
| | Median (%) | NaCl+Pov.Io | Heparine | Activ-Prot C |
| NaCl+Pov.Io | -7.61 | --- | N.S | 0.009 |
| Heparine | 34.30 | --- | --- | 0.015 |
| Activ-Prot C | -21.66 | --- | --- | --- |
| Overall Sig. | 0.009 | | | |

There is statistically significant difference between the 3 therapies of percentage change from baseline to 7^{th} day of the depth of necrosis+ Zone of cellular damage ( p=0.009). Pair wise comparisons indicated statistically significant difference between activated Protein-C and heparine, betadine respectively.

| | Baseline-13^{th} day | | | |
|---|---|---|---|---|
| | Median (%) | NaCl+Pov.Io | Heparine | Activ-Prot C |
| NaCl+Pov.Io | 19.29 | --- | N.S | 0.009 |
| Heparine | 11.83 | --- | --- | 0.002 |
| Activ-Prot C | -43.37 | --- | --- | --- |
| Overall Sig. | 0.006 | | | |

There is statistically significant difference between the 3 therapies of percentage change from baseline to 13^{th} day of the depth of necrosis+ Zone of cellular damage ( p=0,006 ) . Pair wise comparisons indicated statistically significant difference between activated protein C and heparin , betadine respectively.

| | Baseline-16^{th} day | | | |
|---|---|---|---|---|
| | Median (%) | NaCl+Pov.Io | Heparine | Activ-Prot C |
| NaCl+Pov.Io | 26.90 | --- | N.S | 0.05 |
| Heparine | 7.44 | --- | --- | 0.02 |
| Activ-Prot C | -61.81 | --- | --- | --- |
| Overall Sig. | 0.019 | | | |

There is statistically significant difference between the 3 therapies of percentage change from baseline to 16^{th} day of the width of necrosis+ Zone of cellular damage ( p=0,019). Pair wise comparisons indicated statistically significant difference between activated Protein-C and heparine, betadine respectively.

| | Baseline-20^{th} day | | | |
|---|---|---|---|---|
| | Median (%) | NaCl+Pov.Io | Heparine | Activ-Prot C |
| NaCl+Pov.Io | 59.90 | --- | 0.002 | 0.002 |
| Heparine | -28.68 | --- | --- | 0.02 |
| Activ-Prot C | -79.75 | --- | --- | --- |
| Overall Sig. | <0.0005 | | | |

There is statistically significant difference between the 3 therapies of percentage change from baseline to 20^{th} day of the depth of necrosis+ Zone of cellular damage (p<0, 0005) Pairwise comparisons indicated statistically significant difference between all groups

### EXAMPLE 2

The present invention discloses for the first time the use of activated protein C in a clinical trial in non-septic burned patients. In these patients the activated protein C treated group demonstrated statistical improvement in organ function, decreased mortality, and smaller hospitalization time as compared to the placebo group.

Twelve male patients aged 18-56 years of age, received activated protein C upon arrival in a burn unit the last 24 months. Criteria to join the study were the following: Absence of concomitant injuries that interfered with haemorrhage (car accidents with injuries to internal organs as spleen-stomach- kidneys, haemothorax, fractures of long bones, cerebral haemorrhage etc,) presence of partial thickness bums of more of 30% TBSA, burn injury max. 24 hours prior to admission, ABSI (Abbreviated Burn Severity Index Tobiansen) bellow 11 and above 7.

The patients received 24µg/kg/hr of activated protein C upon arrival, which was between 6 and 24 hours postburn but always during the resciscitascion period. During the period of the trial, the survival rate of the above patients reached 80% while the overall survival rate of 53 patients treated in the above Intensive Care Unit with similar ABSI score was around 65%.

Thus the present invention establishes methods utilizing activated protein C in the treatment of partial thickness burns, through the protective action of activated protein C in the survivability of the Zone of Stasis of burns if we administer the drug during the first 48 hours post burn.

## Claims

1. Use of continuous iv administration of activated protein -C and topical bactericidal agent for the manufacture of a medicament for treating partial thickness burns of moderate to severe size up to seven post burn day by protecting and reducing cellular damage in the zone of stasis.

2. The use according to Claim 1 of continues IV administration of activated protein C and topical bactericidal agent for treating partial thickness burns of moderate to severe size during the first 48 hour post burn period by protecting and reducing cellular damage in the zone of stasis.

3. The use according to Claim 2 of continues IV administration of activated protein C at a rate of 24µg/kg/hr for the treatment of partial thickness burns resulting in increased speed of healing of the burned/damaged skin.

4. The use according to Claim 2 of continues IV administration of activated protein C at a rate of 15µg/kg/hr to 30µg/kg/hr for the treatment of partial thickness burns resulting in increased speed of healing of the burned/damaged skin.

## Patentansprüche

1. Verwendung kontinuierlicher intravenöser Verabreichung von aktiviertem Protein C und eines topischen bakteriziden Agens für die Herstellung eines Medikaments zur Behandlung erst- und zweitgradiger Verbrennungen von mittlerem bis schwerem Ausmaß und bis zum siebten Tag nach der Verbrennung, durch den Schutz der und die Verminderung von Zellschäden in der Stasezone.

2. Die Verwendung gemäß Anspruch 1 kontinuierlicher intravenöser Verabreichung von aktiviertem Protein C und eines topischen bakteriziden Agens zur Behandlung erst- und zweitgradiger Verbrennungen von mittlerem bis schwerem Ausmaß während der ersten 48 Stunden nach der Verbrennung, durch den Schutz und die Verminderung von Zellschäden in der Stasezone.

3. Die Verwendung gemäß Anspruch 2 kontinuierlicher intravenöser Verabreichung von aktiviertem Protein C in einer Dosis von 24µg/kg/h zur Behandlung erst- und zweitgradiger Verbrennungen mit dem Ergebnis einer schnelleren Heilung der verbrannten/geschädigten Haut.

4. Die Verwendung gemäß Anspruch 2 kontinuierlicher intravenöser Verabreichung von aktiviertem Protein C in Dosen von 15µg/kg/h bis 30µg/kg/h zur Behandlung erst- und zweitgradiger Verbrennungen mit dem Ergebnis einer schnelleren Heilung der verbrannten/geschädigten Haut.

## Revendications

1. L'utilisation de l'administration IV continue de la protéine C activée et l'utilisation d'un agent bactéricide topique pour la production d'un médicament destiné à traiter les brûlures à épaisseur partielle à étendue modérée ou sévère jusqu'à sept jours post-brûlures et à protéger et réduire les lésions cellulaires dans la zone de stase.

2. L'utilisation selon la revendication 1 de l'administration IV continue de la protéine C activée et d'un agent bactéricide pour le traitement des brûlures à épaisseur partielle à étendue modérée ou sévère pendant les 48 premières heures post-brûlure visant à protéger et réduire les lésions cellulaires dans la zone de stase.

3. L'utilisation selon la revendication 2 de l'administration IV continue de la protéine C activée à 24µg/kg/h pour traiter les brûlures à épaisseur partielle amenant une cicatrisation plus rapide de la peau brûlée/atteinte.

4. L'utilisation selon la revendication 2 de l'administration IV continue de la protéine C active à 15µg/kg/h à 30pg/kg/h pour le traitement des brûlures à épaisseur partielle se traduisant par une cicatrisation plus rapide de la peau brûlée/endommagée.
